(19) Europäisches Patentamt
European Patent Office
Office européen des brevets

(11) Veröffentlichungsnummer: **0 131 942**
**B1**

# (12) EUROPÄISCHE PATENTSCHRIFT

(45) Veröffentlichungstag der Patentschrift: **11.11.87**

(51) Int. Cl.[4]: **C 07 H 15/24**, A 61 K 31/70// C12P19/56

(21) Anmeldenummer: **84108305.8**

(22) Anmeldetag: **14.07.84**

## (54) Anthracyclin-Derivate, ein Verfahren zu ihrer Herstellung und ihre Verwendung als Cytostatika.

(30) Priorität: **19.07.83 DE 3325957**

(43) Veröffentlichungstag der Anmeldung: **23.01.85 Patentblatt 85/04**

(45) Bekanntmachung des Hinweises auf die Patenterteilung: **11.11.87 Patentblatt 87/46**

(84) Benannte Vertragsstaaten: **AT BE CH DE FR GB IT LI LU NL SE**

(56) Entgegenhaltungen:
**EP-A- 0 110 155**
**EP-A- 0 131 181**
**JOURNAL OF ANTIBIOTICS, Band 36, Nr. 5, Mai 1983, Seiten 83-65, Tokyo, JP; "Taurimycin A, Taurimycin B"**
**JOURNAL OF ANTIBIOTICS, Band 29, Nr. 12, Dezember 1976, Seiten 76-116; "Beta-Rhodomycins S-1b, S-2, S-3, S-4"**
**JOURNAL OF ANTIBIOTICS, Band 36, Nr. 8, August 1983, Seiten 1080-1083, Tokyo, JP; "New antitumor antibiotics, ditrisarubicins A, B and C"**

(73) Patentinhaber: **HOECHST AKTIENGESELLSCHAFT**
**Postfach 80 03 20**
**D-6230 Frankfurt am Main 80 (DE)**

(72) Erfinder: **Huber, Gerhard, Dr.**
**In den Bleichwiesen 2**
**D-6233 Kelkheim (Taunus) (DE)**
Erfinder: **Berscheid, Hans Gerd, Dr.**
**Rheinlandstrasse 21**
**D-6231 Schwalbach (DE)**
Erfinder: **Fehlhaber, Hans-Wolfram, Dr.**
**Thomas-Mann-Strasse 5a**
**D-6270 Idstein/Taunus (DE)**
Erfinder: **Kraemer, Hans Peter, Dr.**
**Birkenweg 16**
**D-3550 Marburg (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Anthracyclin-Derivate der Formel I

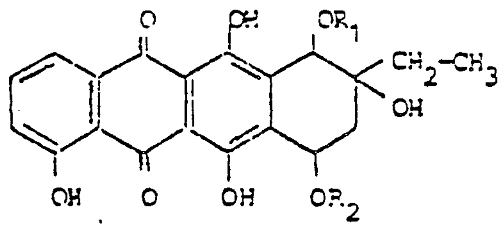

(I)

worin $R_1$ und $R_2$ verschieden sind und den Zuckerrest Roa(L-Rhodosamin) der Formel

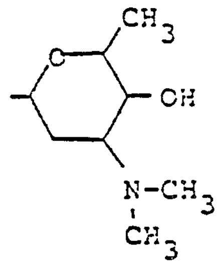

oder die Zuckerkombination Roa—dF=CinB (dF = 2-Desoxy-L-fucose ; CinB = L-Cinerulose B) der Formel

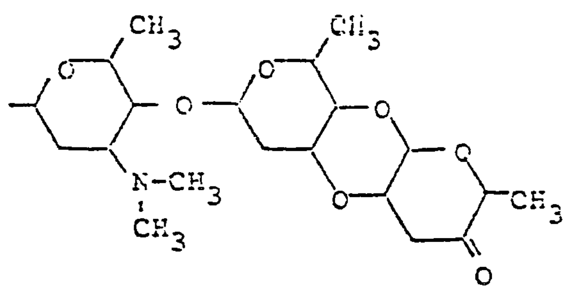

darstellen, sowie ihre pharmazeutisch unbedenklichen Säureadditionssalze.

Die Erfindung betrifft ferner ein Verfahren zur Herstellung der genannten Verbindungen der Formel I, das dadurch gekennzeichnet ist, daß man den Rückstand, der gemäß DE-A-33 230 250 durch Fermentation des Mikroorganismus-Stammes Y-11472 (hinterlegt am 24.5.1983 unter der Eingangsnummer DSM 2658 bei der « Deutsche Sammlung von Mikroorganismen ») und Extraktion des Mycels und des Kulturfiltrats mit einem organischen Lösungsmittel hergestellt wird, mit einer verdünnten starken oder mittelstarken Säure behandelt.

Die so hergestellten Verbindungen zeichnen sich durch hohe cytostatische Wirksamkeit aus.

Gemäß der obengenannten deutschen Patentanmeldung wird der Stamm Streptomyces Y-11472 in einem Nährmedium auf übliche Weise fermentiert, wobei Temperaturen von ca. 24-40 °C, ein pH von 6,5-8,5 und aerobe Bedingungen eingehalten werden. Aus dem Mycel werden die Anthracyclin-Verbindungen durch Extraktion z. B. mit wäßrigem Aceton bei einem pH von 3,5 extrahiert, das Aceton entfernt und die wäßrige Phase bei einem pH von 7,5 mit Ethylacetat extrahiert. Die Kulturflüssigkeit extrahiert man bei einem pH-Wert von 7,5, vorzugsweise mit Ethylacetat. Die Ethylacetatextrakte aus dem Mycel und dem Kulturfiltrat werden vereinigt und ergeben zur Trockne eingedampft einen rohen Rückstand. Man kann nun den so erhaltenen Rückstand erfindungsgemäß einer Säurebehandlung unterziehen. Vorzugsweise arbeitet man jedoch den Rückstand weiter auf wie ebenfalls in der genannten deutschen Patentanmeldung beschrieben.

Demzufolge wird der rohe Rückstand in Toluol gelöst und mit einem Acetatpuffer (pH 3,5) extrahiert, wobei man eine Toluolphase und eine wäßrige Phase erhält. Die wäßrige Phase wird weiter aufgearbeitet in der folgenden Weise : Nach Einstellen des pH auf 7,5 wird wiederum mit Ethylacetat extrahiert und die Ethylacetatphase konzentriert, wobei man ein sogenanntes Cytorhodin-Rohgemisch erhält. Gemäß der genannten deutschen Patentanmeldung können aus diesem Rohgemisch auf chromatographischem Wege Anthracyclin-Derivate mit cytostatischer Wirksamkeit gewonnen werden.

Es wurde nun überraschenderweise gefunden, daß man zu neuen, ebenfalls cytostatisch wirksamen

Anthracyclinen gelangt, wenn man den oben beschriebenen rohen Rückstand oder das sogenannte Cytorhodin-Rohgemisch mit einer verdünnten starken oder mittelstarken Säure behandelt. Als Säuren eignen sich z. B. verdünnte Salzsäure, Schwefelsäure, Phosphorsäure, Ameisensäure, Essigsäure, Trifluoressigsäure und andere Säuren, gegebenenfalls auch nach Zusatz eines mit Wasser mischbaren organischen Lösungsmittels z. B. Methanol oder Aceton. Man kann die Säurebehandlung auch mit stark sauren Ionenaustauschern in der H-Form wie z. B. Dowex 50 × 8 [$H^+$] durchführen. In diesem Fall muß das gewünschte Produkt durch Elution vom Ionenaustauscher, z. B. mit Salzlösungen, gewonnen werden.

Die Säurebehandlung wird bei Raumtemperatur oder leicht erhöhter Temperatur, vorzugsweise 37 °C, durchgeführt. Die Reaktionszeit kann 0,5 bis mehrere Stunden betragen. Nach erfolgter Säurebindung werden die gewünschten Verbindungen durch Extraktion aus dem Reaktionsmedium isoliert und durch Säulenchromatographie, vorzugsweise an Kieselgel, getrennt.

Man erhält durch das erfindungsgemäße Verfahren zum Beispiel die folgenden Verbindungen :

Cytorhodin S

Rote, amorphe Substanz, leicht löslich in Methanol, Ethylacetat, Chloroform und Toluol, unlöslich in Wasser und Hexan.

Dünnschichtchromatographie : Kieselgel 60 $F_{254}$ « Merck »
System B : Chloroform/Methanol/Eisessig/Wasser
70 : 20 : 10 : 2, RF = 0,67
UV-Maxima : 236, 252 (Sch), 293, 497, 563 nm in Methanol/10 % 1N HCl
NMR-Spektrum : Abb. 1
$C_{48}H_{64}N_2O_{17}$, M ber. 940 (FAB-MS bestätigt)
Das Cytorhodin S besitzt die Formel I, worin $R_1$ —Roa und $R_2$ —Roa—dF=CinB bedeutet.

Cytorhodin T

Rote, amorphe Substanz, leicht löslich in Methanol, Ethylacetat, Chloroform und Toluol, unlöslich in Wasser und Hexan.

Dünnschichtchromatographie : Kieselgel 60 $F_{254}$ « Merck »
System B : Chloroform/Methanol/Eisessig/Wasser
70 : 20 : 10 : 2, RF = 0,53
UV-Maxima : 236, 252 (Sch), 293, 496, 563 nm in Methanol/10 % 1N HCl
NMR-Spektrum : Abb. 2
$C_{48}H_{64}N_2O_{17}$, M ber. 940 (FAB-MS bestätigt)
Das Cytorhodin-T besitzt die Formel I, worin $R_1$ —Roa—dF=CinB und $R_2$ —Roa bedeutet.

Neben den neuen Cytorhodinen wird auch das schon bekannte β-Rhodomycin II (Rhodomycin A, H. Brockmann et al., Naturwiss. 37, 492, 1950 ; ibid 42, 154, 1955) gebildet (Formel I, worin $R_1$ und $R_2$ jeweils —Roa bedeuten.)

Die erfindungsgemäßen Cytorhodine zeichnen sich durch eine hohe cytostatische Wirksamkeit aus und ergaben im Zellproliferationstest an L 1210 — Leukämiezellen folgende Wirkung :

| | $IC_{50}$ (μg/ml) |
|---|---|
| Cytorhodin S | $3 \times 10^{-4}$ |
| Cytorhodin T | $2 \times 10^{-3}$ |

Die Hemmwirkung wird folgendermaßen bestimmt/Zellproliferationstest) :

L 1210 Zellen in exponentieller Wachstumsphase (5 × $10^3$/ml in RPMI 1640) werden in einer Mikrotiterplatte 72 Stunden mit unterschiedlichen Konzentrationen der Testsubstanz inkubiert (37 °C, 5 % $CO_2$, 95 % relative Luftfeuchte). Kontrollen bestehen aus Zellen, die lediglich mit frischem Medium inkubiert werden. Alle Bestimmungen werden als 4-fache Bestimmungen durchgeführt. Nach 65 Stunden werden 50 μl C14-Thymidin (55,5 kBq/ml oder 1,5 μCi/ml) zugegeben, um die DNA der Zelle radioaktiv zu markieren. Nach 7 Stunden Inkubation werden die Zellen abgesaugt, die DNA mit 5 %iger Trichloressigsäure gefällt und nacheinander mit Wasser bwz. Methanol gewaschen.

Nach Trocknung bei 50 °C wird die in die DNA eingebaute Radioaktivität nach Zugabe von 5 ml Szintillationsflüssigkeit ermittelt. Die Ergebnisse werden angegeben als Verhältnisse des Szintillationsindex nach Inkubation mit Testsubstanz in Prozent der unbehandelten Kontrolle. Aus den so erhaltenen Meßwerten wird die Dosiswirkungskurve ermittelt und grafisch die $IC_{50}$, d. h. die Konzentration, die unter Testbedingungen den Einbau von radioaktivem Thymidin um 50 % gegenüber der Kontrolle erniedrigt, ermittelt.

Die Herstellung der erfindungsgemäßen Verbindungen wird in den nachfolgenden Beispielen beschrieben :

Beispiel 1

4,5 g roher Cytorhodin-Komplex werden in 250 ml 0,2 n HCl gelöst und 1 Stunde auf 37 °C erwärmt. Die Lösung wird mit gekühlter 1n NaOH auf pH 7,5 eingestellt und 4mal mit je 300 ml Chloroform extrahiert. Der Chloroformextrakt wird im Vakuum zur Trockne eingedampft. Der Rückstand (2,1 g) wird in 30 ml eines Gemisches von Chloroform/Methanol/Eisessig/Wasser 50 : 50 : 5 : 2 (System A) gelöst und auf eine Säule mit 200 ml Kieselgel 60 « Merck », 40-63 µm, in System A aufgeschlämmt, aufgetragen und mit dem gleichen System A eluiert. Nach 100 ml Vorlauf werden Fraktionen je 10 ml aufgefangen und im Dünnschichtchromatogramm (Kieselgel 60 $F_{254}$ « Merck », System B : Chloroform/Methanol/Eisessig/Wasser 70 : 20 : 10 : 2) analysiert. Die aktiven Substanzen erscheinen in folgenden Fraktionen :

18-31 Cytorhodin S und T
45-67 β-Rhodomycin II

Die Fraktionen 18-31 werden vereinigt und bis zur Abscheidung der Chloroformphase mit einer 5 %igen wäßrigen Lösung von $Na_2HPO_4$-Lösung versetzt, die Chloroformphase mit je 1 Volumen $Na_2HPO_4$-Lösung und Wasser gewaschen, über wasserfreiem Natriumsulfat getrocknet, im Vakuum eingeengt und mit Hexan gefällt.

Ausbeute 540 mg Gemisch der Komponenten Cytorhodin S und T.

Beispiel 2

300 mg nach Beispiel 1 erhaltenes Gemisch von Cytorhodin S und T werden in 20 ml System B (siehe Beispiel 1) gelöst und auf eine Säule von 100 ml Kieselgel 60 « Merck », 15-40 µm, im gleichen System aufgeschlämmt, aufgetragen und mit System B eluiert. Nach einem Vorlauf von 100 ml werden die aktiven Fraktionen je 5 ml aufgefangen :

| | |
|---|---|
| 21-32 Cytorhodin S | 160 mg |
| 41-80 Cytorhodin S + wenig T | 35 mg |
| 81-100 Cytorhodin T | 40 mg |

Die vereinigten Fraktionen werden mit einer 5 %igen wäßrigen $Na_2HPO_4$-Lösung bis zur Abscheidung der Chloroformphase versetzt, diese mit je 1 Volumen $Na_2HPO_4$-Lösung und Wasser gewaschen, über wasserfreiem Natriumsulfat getrocknet, im Vakuum eingeengt und mit Hexan gefällt.

Ausbeute :
160 mg reines Cytorhodin S
40 mg reines Cytorhodin T

Beispiel 3

1,1 g Cytorhodin-Komplex werden in 60 ml 0,2 n Ameisensäure gelöst und 24 Stunden auf 37 °C erwärmt. Die Lösung, die laut Dünnschichtchromatogramm die Cytorhodine S und T enthält, wird mit 1n NaOH auf pH 7,5 eingestellt und wie in Beispiel 1 aufgearbeitet.

Beispiel 4

Eine Lösung von 0,5 g Cytorhodin-Komplex in 28 ml $H_2O$ wird unter Rühren mit 0,64 ml Trifluoressigsäure versetzt und anschließend 4 Stunden bei Raumtemperatur stehen gelassen. Die Lösung wird wie in Beispiel 1 und 2 auf Cytorhodin S und T aufgearbeitet.

Die Identifizierung der Komponenten in den vorstehenden Beispielen erfolgte unter den anschließend beschriebenen Meßbedingungen :

Die Protonen-Resonanzspektren ($^1H$-NMR-Spektren) wurden auf einem HX-270 BRUKER Fourier-Transform Kernresonanzspektrometer bei 270 MHZ gemessen. Die Konzentrationen betrugen 2-4 mg/0,5 ml 99,8 % $CDCl_3$ ; die Lösungen wurden sofort nach Herstellung mit 0,1 ml %iger $Na_2CO_3$ 99,5 % $D_2o$ geschüttelt.

Die in den Abbildungen mit einem Stern versehenen Signale rühren her von niedermolekularen Verunreinigungen im ‰ Bereich und von Lösungsmittelresten.

Die Massenspektren wurden auf dem Massenspektrometer MS-902 S, AEI, unter Verwendung einer FAB-(Fast-Atom-Bombardment-)Ionenquelle gemessen. Die Substanzen wurden in einer Matrix von Thioglycerin in die Ionenquelle eingebracht, teilweise unter Zusatz von Ammoniumchlorid.

**Patentansprüche** (für die Vertragsstaaten : BE, CH, DE, FR, GB, IT, LI, LU, NL, SE)

1. Verbindungen der allgemeinen Formel I

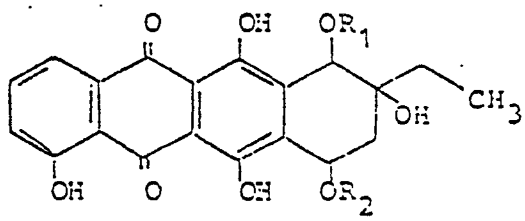

(I)

wobei $R_1$ und $R_2$ verschieden sind und folgende Bedeutung haben können :

—Roa der Formel

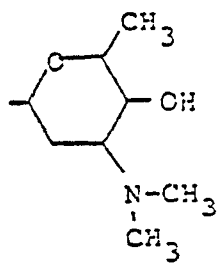

(II)

oder —Roa—dF=CinB der Formel

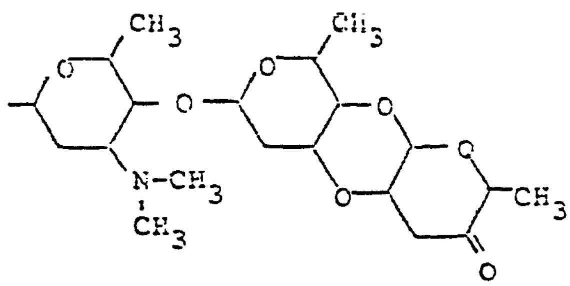

(III)

sowie ihre pharmazeutisch unbedenklichen Säureadditionssalze.

2. Verbindung der Formel I gemäß Anspruch 1, worin $R_1$ —Roa und $R_2$ —Roa—dF=CinB bedeutet.

3. Verbindung der Formel I gemäß Anspruch 1, worin $R_1$ —Roa—dF=CinB und $R_2$ —Roa bedeutet.

4. Verfahren zur Herstellung von Verbindungen gemäß Anspruch 1, dadurch gekennzeichnet, daß man ein Cytorhodin-Rohgemisch, das gemäß DE-A-33 23 025 erhältlich ist, einer Behandlung mit einer verdünnten starken oder mittelstarken Säure bei Raumtemperatur oder leicht erhöhter Temperatur unterwirft und die gewünschten Produkte durch Extraktion und chromatographische Methoden isoliert.

5. Verfahren nach Anspruch 1 dadurch gekennzeichnet, daß man als Säure Salzsäure, Schwefelsäure, Phosphorsäure, Ameisensäure, Essigsäure oder Trifluoressigsäure verwendet.

6. Verfahren nach Anspruch 1, dadurch gekennzeichnet, daß eine Verbindung der Formel I hergestellt wird, worin $R_1$ den Zuckerrest —Roa und $R_2$ die Zuckerkombination —Roa—dF=CinB bedeuten.

7. Verfahren nach Anspruch 1, dadurch gekennzeichnet, daß eine Verbindung der Formel I hergestellt wird, worin $R_1$ die Zuckerkombination Roa—dF=CinB und $R_2$ den Zuckerrest —Roa bedeuten.

8. Arzneimittel, gekennzeichnet durch den Gehalt einer Verbindung gemäß Anspruch 1 und einem pharmazeutisch üblichen Träger.

9. Verwendung einer Verbindung gemäß Anspruch 1 zur Herstellung eines Arzneimittels mit cytostatischer Wirkung.

**Patentansprüche** (für den Vertragsstaat AT)

1. Verfahren zur Herstellung von Verbindungen der allgemeinen Formel I

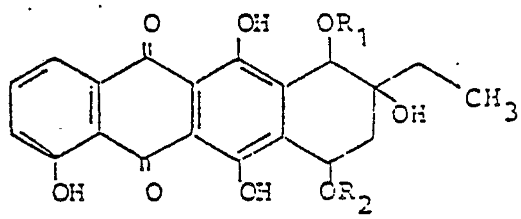

(I)

wobei $R_1$ und $R_2$ verschieden sind und folgende Bedeutung haben können :

—Roa der Formel

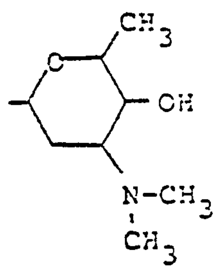

(II)

oder —Roa—dF=CinB der Formel

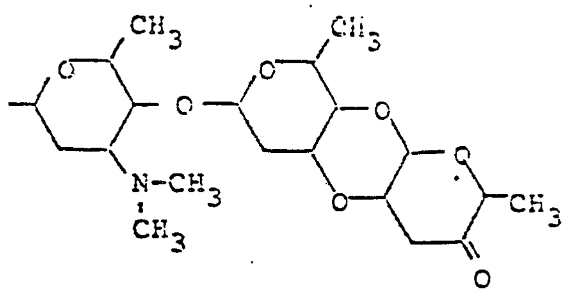

(III)

dadurch gekennzeichnet, daß man ein Cytorhodin-Rohgemisch das gemäß DE-A-33 23 025 erhältlich ist, einer Behandlung mit einer verdünnten starken oder mittelstarken Säure bei Raumtemperatur oder leicht erhöhter Temperatur unterwirft und die gewünschten Produkte durch Extraktion und chromatographische Methoden isoliert.

2. Verfahren nach Anspruch 1 dadurch gekennzeichnet, daß man als Säure Salzsäure, Schwefelsäure, Phosphorsäure, Ameisensäure, Essigsäure oder Trifluoressigsäure verwendet.

3. Verfahren nach Anspruch 1 und 2, dadurch gekennzeichnet, daß eine Verbindung der Formel I hergestellt wird, worin $R_1$ den Zuckerrest —Roa und $R_2$ die Zuckerkombination —Roa—dF=CinB bedeuten.

4. Verfahren nach Anspruch 1 und 2, dadurch gekennzeichnet, daß eine Kombination der Formel I hergestellt wird, worin $R_1$ die Zuckerkombination Roa—dF=CinB und $R_2$ den Zuckerrest —Roa bedeuten.

**Claims** (for the contracting states : BE, CH, LI, DE, FR, GB, IT, LU, NL, SE)

1. A compound of the formula I

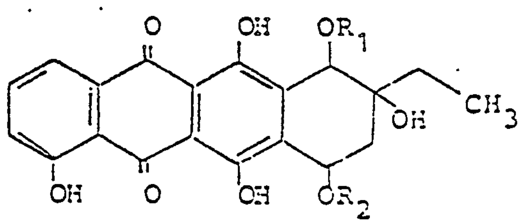

(I)

in which $R_1$ and $R_2$ are different and can have the following meaning :

—Roa of the formula

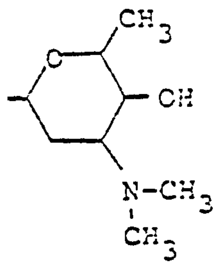

(II)

or —Roa—dF=CinB of the formula

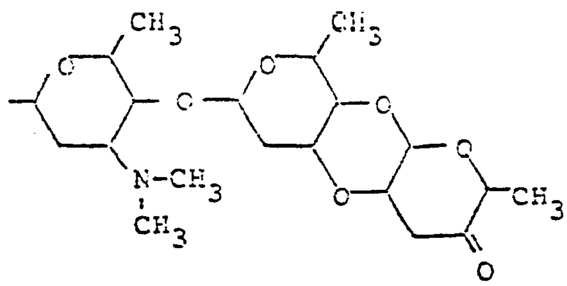

(III)

and the pharmaceutically acceptable acid addition salts thereof.

2. The compounds of the formula I as claimed in claim 1, in which $R_1$ denotes —Roa and $R_2$ denotes —Roa—dF=CinB.

3. The compound of the formula I as claimed in claim 1, in which $R_1$ denotes —Roa—dF=CinB and $R_2$ denotes —Roa.

4. A process for the preparation of a compound according to claim 1 which comprises subjecting a crude cytorhodin mixture which can be obtained according to DE-A-33 23 025 to treatment with a dilute strong or moderately strong acid at room temperature or slightly elevated temperature and isolating the desired product by extraction and chromatographic methods.

5. The process as claimed in claim 1, wherein hydrochloric acid, sulfuric acid, phosphoric acid, formic acid, acetic acid or trifluoroacetic acid is used as the acid.

6. The process as claimed in claim 1, wherein a compound of the formula I in which $R_1$ denotes the sugar residue —Roa and $R_2$ denotes the sugar combination —Roa—dF=CinB is prepared.

7. The process as claimed in claim 1, wherein a compound of the formula I in which $R_1$ denotes the sugar combination —Roa—dF=CinB and $R_2$ denotes the sugar residue —Roa is prepared.

8. A pharmaceutical composition which comprises as active ingredient a compound of the formula I as defined in claim 1 in association with a pharmaceutically acceptable carrier.

9. Use of a compound as defined in claim 1 for the preparation of a medicament having cytostatic activity.

**Claims** (for the Contracting State AT)

1. A compound of the formula I

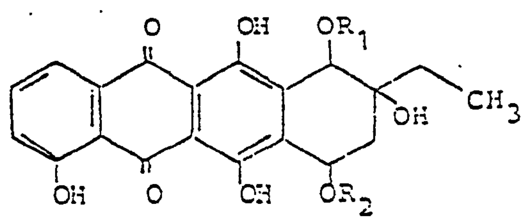

(I)

in which $R_1$ and $R_2$ are different and can have the following meaning :

—Roa of the formula

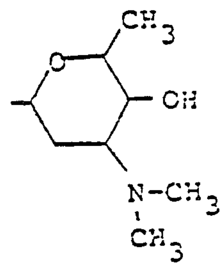

(II)

or —Roa—dF=CinB of the formula

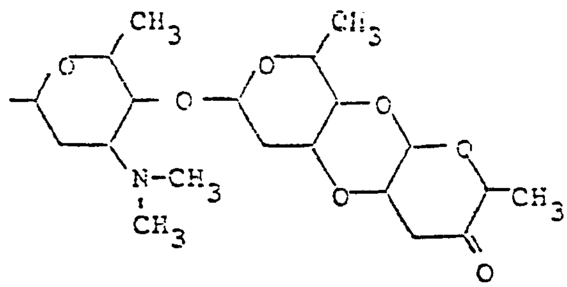

(III)

and the pharmaceutically acceptable acid addition salts thereof which comprises subjecting a crude cytorhodin mixture which can be obtained according to DE-A-33 23 025 to treatment with a dilute strong or moderately strong acid at room temperature or slightly elevated temperature and isolating the desired product by extraction and chromatographic methods.

2. The process as claimed in claim 1, wherein hydrochloric acid, sulfuric acid, phosphoric acid, formic acid, acetic acid or trifluoroacetic acid is used as the acid.

3. The process as claimed in claim 1, wherein a compound of the formula I in which $R_1$ denotes the sugar residue —Roa and $R_2$ denotes the sugar combination —Roa—dF=CinB is prepared.

4. The process as claimed in claim 1, wherein a compound of the formula I in which $R_1$ denotes the sugar combination Roa—dF=CinB and $R_2$ denotes the sugar residue —Roa is prepared.

**Revendications** (pour les Etats contractants : BE, CH, LI, DE, FR, GB, IT, LU, NL, SE)

1. Composé de formule générale I

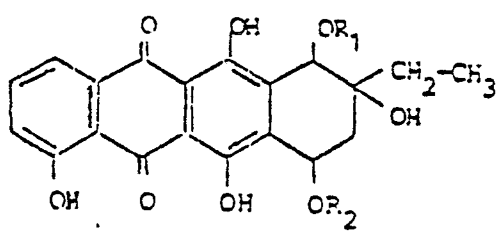

(I)

dans laquelle $R_1$ et $R_2$ sont différents et peuvent avoir la signification suivante :

—Roa de formule

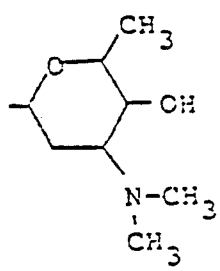

(II)

où —Roa—dF=CinB de formule

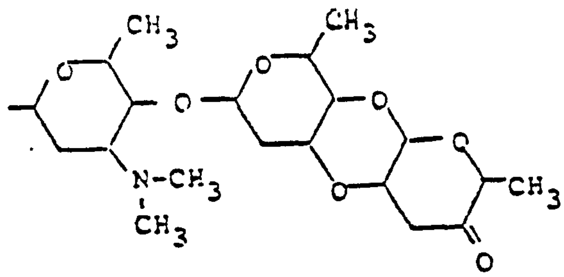

(III)

ainsi que leurs sels d'addition à un acide pharmaceutiquement acceptable.

2. Composé de formule I selon la revendication 1 dans lequel $R_1$ signifie —Roa et $R_2$ signifie —Roa—dF=CinB.

3. Composé de formule I selon la revendication 1 dans laquelle $R_1$ signifie —Roa—dF=CinB et $R_2$ signifie —Roa.

4. Procédé de préparation de composés selon la revendication 1, caractérisé en ce que l'on soumet un mélange brut de cytorhodines, qui peut être obtenu suivant DE-A-33 23 025, à un traitement avec un acide fort ou moyennement fort dilué à la température ambiante ou à une température légèrement plus élevée et en ce que l'on sépare les produits désirés par extraction et par des méthodes chromatographiques.

5. Procédé selon la revendication 1 caractérisé en ce que l'on emploie comme acides l'acide chlorhydrique, l'acide sulfurique, l'acide phosphorique, l'acide formique, l'acide acétique ou l'acide trifluoroacétique.

6. Procédé selon la revendication 1 caractérisé en ce que l'on prépare un composé de formule I dans laquelle $R_1$ désigne le radical de sucre —Roa et $R_2$ désigne la combinaison de sucre —Roa—dF=CinB.

7. Procédé selon la revendication 1 caractérisé en ce que l'on prépare un composé de formule I dans laquelle $R_1$ désigne la combinaison de sucre —Roa—dF=CinB et $R_2$ désigne le radical de sucre —Roa.

8. Médicament caractérisé en ce qu'il contient un composé selon la revendication 1 et un support pharmaceutiquement usuel.

9. Emploi d'un composé selon la revendication 1 pour la préparation d'un médicament à action cytostatique.

**Revendications** (pour l'Etat contractant AT)

1. Procédé de préparation de composés de formule générale I

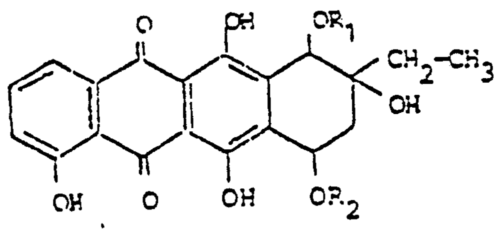

(I)

dans laquelle $R_1$ et $R_2$ sont différents et peuvent avoir la signification suivante :

—Roa de formule

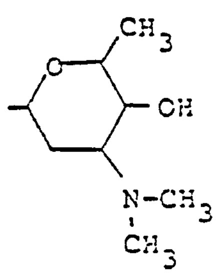

(II)

où —Roa—dF=CinB de formule

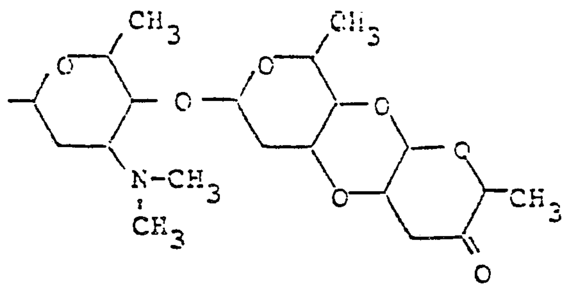

(III)

caractérisé en ce que l'on soumet un mélange brut de cytorhodine, qui peut être obtenu suivant DE-A-33 23 025, à un traitement par un acide fort ou moyennement fort dilué à la température ambiante ou à une température légèrement plus élevée et en ce que l'on isole les produits désirés par extraction et par des méthodes chromatographiques.

2. Procédé selon la revendication 1 caractérisé en ce que l'on emploie comme acide l'acide chlorhydrique, l'acide sulfurique, l'acide phosphorique, l'acide formique, l'acide acétique ou l'acide trifluoroacétique.

3. Procédé selon les revendications 1 et 2 caractérisé en ce que l'on prépare un composé de formule I dans laquelle $R_1$ désigne le radical de sucre —Roa et $R_2$ désigne la combinaison de sucre —Roa—dF=CinB.

4. Procédé selon les revendications 1 et 2 caractérisé en ce que l'on prépare un composé de formule I dans laquelle $R_1$ désigne la combinaison de sucre —Roa—dF=CinB et $R_2$ désigne le radical de sucre —Roa.

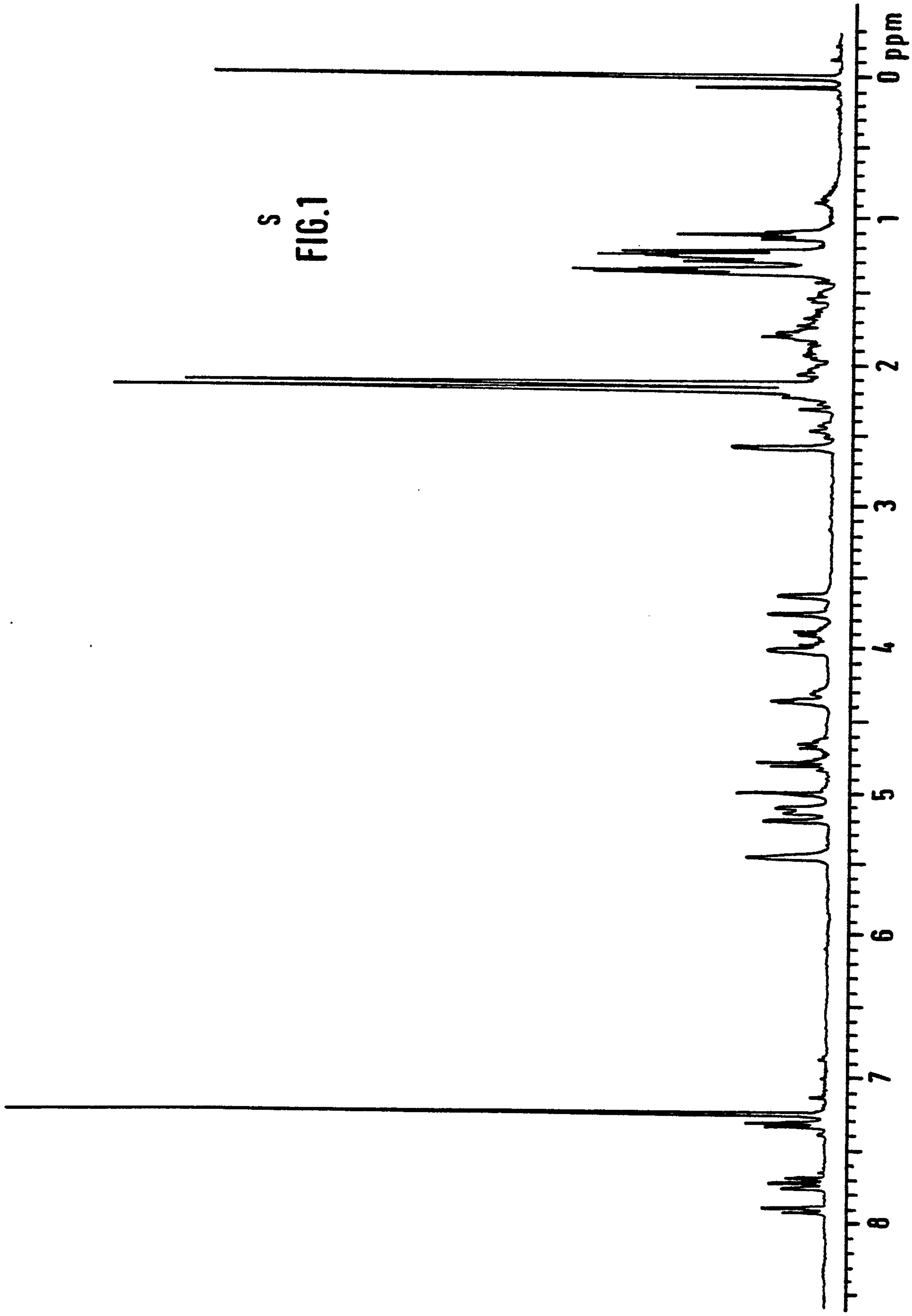


S
FIG.1

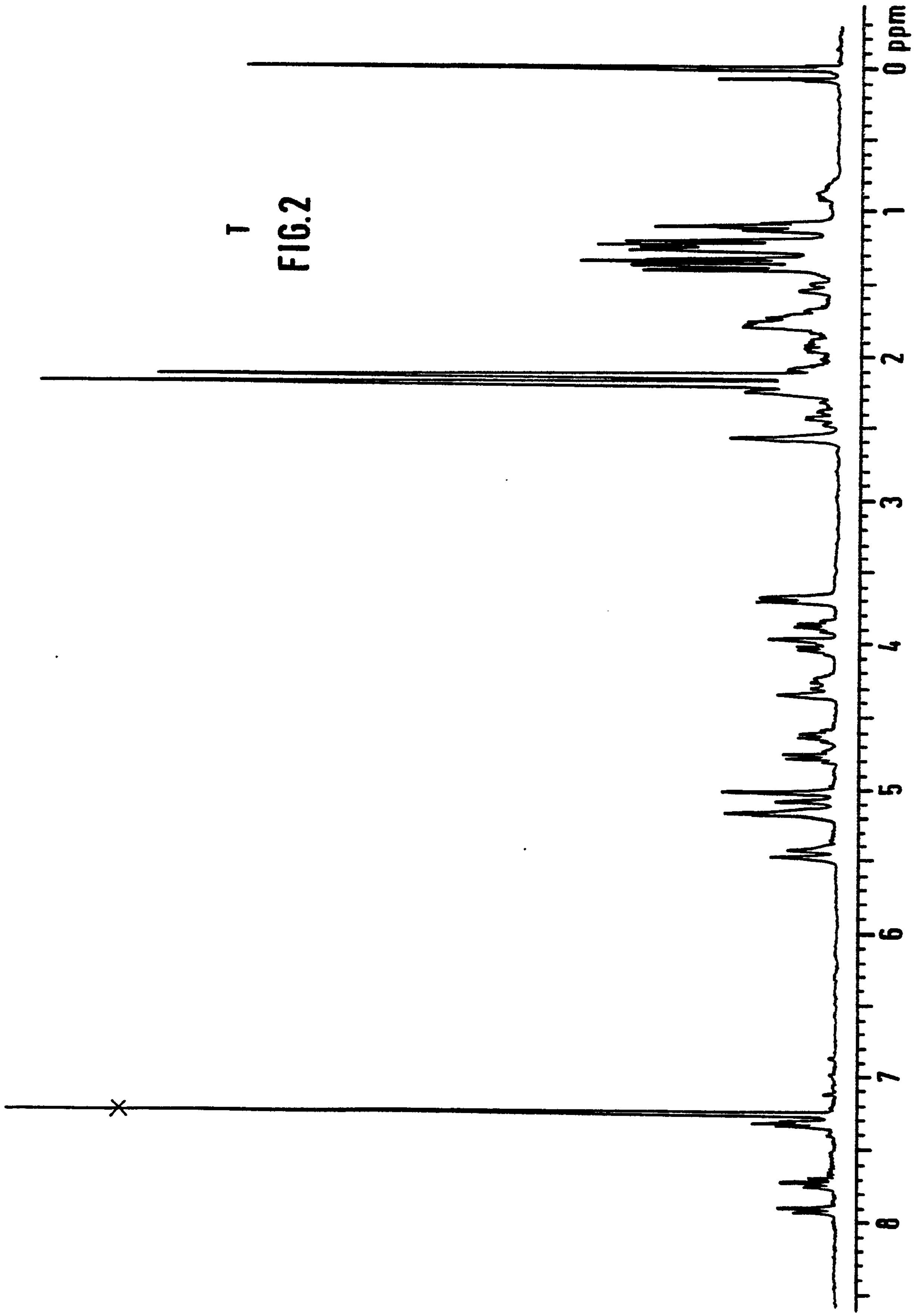


T

FIG.2